# EUROPEAN PATENT SPECIFICATION

(11) **EP 2 761 271 B1**
(45) Date of publication and mention of the grant of the patent: **24.10.2018**
(21) Application number: 12766107.2
(22) Date of filing: 28.09.2012
(51) Int. Cl.: G01N 21/35

(54) **METHOD FOR MEASURING A BIOLOGICAL VALUE OF A LIVER**
VERFAHREN ZUM MESSEN EINES BIOLOGISCHEN WERTS EINER LEBER
PROCÉDÉ POUR MESURER LA VALEUR BIOLOGIQUE D'UN FOIE

(30) Priority: 29.09.2011 EP 11306256
(43) Date of publication of application: 06.08.2014
(73) Proprietor: INSERM - Institut National de la Santé et de la Recherche Médicale, 75013 Paris (FR); SYNCHROTRON SOLEIL, 91190 St. Aubin (FR); Université Paris-Sud 11, 91405 Orsay Cedex (FR)
(72) Inventor: LE NAOUR, François, F-94800 Villejuif (FR); DUMAS, Paul, F-91190 Gif sur Yvette (FR); GUETTIER, Catherine, F-94800 Villejuif (FR)
(74) Representative: Cabinet Plasseraud
(86) International application number: PCT/EP2012/069276
(87) International publication number: WO 2013/045670

(56) References cited:
- EP-A1- 2 302 359
- US-A- 5 038 039
- US-A1- 2008 071 154

## Description

### FIELD OF THE INVENTION

The present invention relates to a method for measuring a level of lipids in a liver.

### BACKGROUND OF THE INVENTION

Fatty liver disease encompasses a wide spectrum of clinical conditions such as alcoholism, drug intake, small-bowel by-pass surgery or metabolic syndrome and is also frequently associated with chronic hepatitis C. Non alcoholic fatty liver disease (NAFLD) known to be associated with obesity, insulin resistance, diabetes, hypertriglyceridemia, arterial hypertension in the metabolic syndrome is probably the most common cause of chronic liver disease in Western countries. Fatty liver disease is also a potential long-term complication of liver transplantation.

The clinical-histological spectrum of NAFLD includes non-alcoholic fatty liver (NAFL), non-alcoholic steatohepatitis (NASH) and steatofibrosis. Estimates obtained from clinical series, autopsy studies, and convenience samples of the general population suggest that up to 30 % of individuals in Western countries could have NAFLD (Neuschwander-Tetri, 2003, Torres, 2008). NASH has been estimated to affect 5%-7% of the general population. NASH can progress to cirrhosis in up to 15% of patients with a potential risk of progression to liver failure and hepatocellular carcinoma. Thus, NAFLD is now recognized as a major cause of cirrhosis in Western countries.

Whereas steatosis is reversible and currently considered innocuous in its pure form, some patients with pure steatosis can progress to NASH (Wong, 2010) and most importantly, some patients with NASH can also progress to hepatocellular carcinoma bypassing the stage of cirrhosis.

The mechanisms responsible for the evolution from steatosis to steatohepatitis are not fully understood thus preventing prognosis of the disease at an individual level.

Despite the major public health concern of NAFLD, it is currently impossible to identify, at an early stage, patients who will progress to NASH and fibrosis from patients who will remain at the steatosis stage.

Thus, there is an urgent need of diagnostic and prognosis markers in fatty liver diseases, in particular for identifying steatosis level and predicting if steatosis will evolve to steatohepatitis.

Quantifying the level of steatosis is also a major issue in liver transplantation (LT).

Indeed, there are a significant number of cases of liver transplantation leading to primary non-function or delayed function of the graft mainly due to the poor quality of the graft and especially the presence of steatosis.

Steatosis is one of the most important factors affecting liver allograft function. Although steatosis can regress within weeks after liver transplantation, early functional recovery and regenerative capacity are significantly impaired with steatotic allografts, mostly because of more severe ischemia-reperfusion injury.

Steatosis of the graft is not only a cause of primary graft dysfunction but also a source of long-term poorer evolution of the graft. In patients transplanted for HCV cirrhosis, the survival of the grafts was inversely proportional to donor liver steatosis (72% at 3 years post-OLT in the absence of steatosis versus 43% with moderate steatosis) and HCV recurrence is more frequent and earlier in recipients of moderately and severely steatotic livers. Fibrosis evolution is higher when graft steatosis is higher than 30% (Briceno, 2009). The issue is that there is no objective and quantifiable marker for graft quality control. The only control performed on the liver from donor is based on frozen section examination. The evaluation of the level of steatosis on histological sections is strongly observer-dependent and not reproducible (El Badry, 2009).

Quantifying steatosis is the all more important since a major limitation of liver transplantation is the shortage of grafts. This situation led transplantation teams to the use of marginal grafts from expanded criteria donors. One of these expanded criteria is the presence of steatosis.

The use of grafts with moderate steatosis (30%-60%) remains a challenging issue. In this group, the incidence of primary non-functional may reach 15%, and the rate of delayed graft function approaches 35%. Because of the combination of expanded criteria, liver biopsy quantification of degree of steatosis should be as accurate as possible.

The lack of grafts has also led to using organs obtained on non-heart-beating donors who have just deceased. Liver transplantation from non-heart-beating donors implies a particular and standardized strategy. Recently, a specific protocol of liver harvesting and transplant from non-heart-beating donors was drafted by French transplantation physicians invited by the Agency of the Biomedicine. In this protocol, important criteria were suggested, in particular the systematic histological examination of a biopsy with recommendation to select livers exhibiting no more than 20% steatosis. This drastic recommendation contrasts with the incapacity of usual histological methods to rigorously provide a non-biased assessment of steatosis.

Indeed, nowadays, the usual histological methods used to evaluate the quality of a graft before LT are only qualitative. They are based on the optical evaluation of hepatocytes containing fat inclusions on a tissue section by a physician. El Badry et al. (2009) have shown that this evaluation is highly dependent on the physician interpretation and therefore subjective.

Furthermore, this qualitative approach is not adapted in case of microsteatosis because of the extremely small size of vacuoles that make them difficult to detect by a physician.

The only reliable method to quantitatively and objectively evaluate steatosis in a liver is to chemically extract the lipids from a sample of the graft and to determine the content of lipids by gas-phase chromatography coupled to mass spectrometry (Rebouissou et al., 2007). This method is lengthy since it takes about two days. Furthermore, it is inappropriate in case of LT, wherein a decision of the surgeon to proceed to graft must be taken in 15 minutes maximum from the time the liver biopsy from the liver graft arrives in the service of pathology for histological examination.

US Patent Number 5,038,039 discloses a method for detecting the presence of anomalies in biological tissues using infrared spectroscopy. In this method, a beam of infrared light is directed to a sample of biological tissue, and the anomaly is detected at at least one range of frequencies by determining whether changes in infrared absorption have occurred compared to a healthy, control tissue sample.

### SUMMARY OF THE INVENTION

The Applicant has now found that the use of infrared radiation allowed the quantitative and objective evaluation of a level of lipids in a liver. This evaluation allows a quick and reliable diagnosis of this liver, in particular of it ability to be grafted.

More precisely, the inventors have discovered that the infrared signal of a sample of liver at a specific wave number range can quantitatively provide a level of lipids of a liver, thereby providing accurate quantitative data about the liver and thus a fast diagnostic of clinical relevance.
A subject of the present invention is therefore a method for measuring a level of lipids Vb in a liver wherein said method comprises the steps of:
a/ dividing a solid sample of liver into two or more portions, pᵢ,
b/ utilizing an infrared spectrometer to apply an infrared radiation having at least a first wave number range between 2800 cm⁻¹ and 3000 cm⁻¹ to each of the two or more portions, pᵢ, of said sample of said liver,
c/ detecting the intensity of the radiation after it has passed through each of the two or more portions, pᵢ, and generating a signal related to each detected intensity,
d/ processing the generated signals to calculate an average value Vₐ; by:
   - calculating, for each of the two or more portions, pᵢ, a first value V_{w1pi} related to the first wave number range, and
   - calculating the average value Vₐ based on the first values of all the portions,
      or
   - calculating the mean of the generated signals for each portion pᵢ to generate an average signal, and
   - calculating a first average value V_{w1a} related to the average signal at the first wave number range, wherein V_{w1a} is equal to the average value Vₐ, and
e/ comparing said average value Vₐ to a standard to obtain the level of lipids Vb.

In certain embodiments, in the method according to the invention, the step of processing the generated signals to calculate an average value comprises the steps of:
- calculating, for each of the two or more portions, pᵢ, a first value V_{w1pi} related to the first wave number range, and
- calculating an average value Vₐ based on the first values of all the portions.

In certain embodiments, the steps of processing the generated signals to calculate an average value comprises the steps of:
- calculating the mean of the generated signals for each portion pᵢ to generate an average signal, and
- calculating a first value V_{w1a} related to the average signal at the first wave number range, wherein Vw1a is equal to the average value Va.

In certain embodiments:
- the infrared radiation has further a second wave number range between 1450 cm⁻¹ and 1710 cm⁻¹, and
- the steps of processing the generated signals to calculate an average value Vₐ further comprises the steps of:
   - for each of the two or more portions, pi, calculating a second value V_{w2pi} related to the second wave number range, wherein the average value Va is the average ratio V_{w1pi}/V_{w2pi},
      or
   - calculating the mean of the generated signals for each portion pi to generate an average signal,
   - calculating a second average value V_{w2a} related to the average signal at the second wave number range, wherein the average value Va is the average ratio V_{w1a}/Vw₂ₐ

For example, the step of processing the generated signals to calculate an average value Vₐ may comprise the steps of:
- calculating for each of the two or more portions, pi, a second value V_{w2pi} related to the second wave number range.

Alternatively, the step of processing the generated signals to calculate an average value Vₐ comprises steps of:
- calculating the mean of the generated signals for each portion pi to generate an average signal,
- calculating a second average value V_{w2a} related to the average signal at the second wave number range.

The the second wave number range may be between 1450 cm⁻¹ and 1575 cm⁻¹.

Alternatively, the second wave number range is between 1660 cm⁻¹ and 1710 cm⁻¹.

The present invention also relates to an *in vitro* method for diagnosing a fatty liver comprising the steps of:
- measuring a level of lipids Vb according the method of the present invention and
- comparing the level of lipids Vb to a threshold value,
wherein a level of lipids Vb superior to the threshold value is indicative of a fatty liver.

The present invention also relates to an *in vitro* method for determining the level of steatosis of a liver comprising the steps of:
- measuring a level of lipids Vb according the method of the present invention and
- comparing the level of lipids Vb to threshold values,
wherein:
- a level of lipids Vb between a first threshold value and a second threshold value is indicative of a mild steatosis,
- a level of lipids Vb between the second threshold value and a third threshold value is indicative of a moderate steatosis,
- a level of lipids Vb superior to the third threshold value is indicative of a severe steatosis.

Preferably, the first threshold value is inferior to the second threshold value and the second threshold value is inferior to the third threshold value.

The present invention also relates to an *in vitro* method for determining the risk of developing steatofibrosis, hepatocarcinoma or cirrhosis comprising the steps of:
- measuring a level of lipids Vb according the method of the present invention and
- comparing the level of lipids Vb to threshold values,
wherein:
a level of lipids Vb superior to a first threshold value is indicative of a high risk to develop steatofibrosis,
a level of lipids Vb superior to a second threshold value is indicative of a high risk to develop hepatocarcinoma,
a level of lipids Vb superior to a third threshold value is indicative of a high risk to develop cirrhosis.

The present invention also relates to an *in vitro* method for determining if a liver is suitable to be grafted comprising the steps of:
- measuring a level of lipids Vb according the method of the present invention and
- comparing the level of lipids Vb to threshold values,
wherein
- a level of lipids Vb inferior to a first threshold value is indicative that the liver is suitable to be grafted with a poor risk of non-function.
- a level of lipids Vb between the first and a second threshold value is indicative that the liver is suitable to be grafted with a moderate risk of non-function,
- a level of lipids Vb superior to a third threshold value is indicative that the liver is not suitable to be grafted.

Preferably, the first threshold value is inferior to the second threshold value and the second threshold value is inferior or equal to the third threshold value.

Is also described herein an apparatus for measuring a biological value Vb, such as a level of lipids, in a sample comprising:
- a site for submitting a sample to infrared radiation
- an infrared radiation source for providing a beam of infrared radiation having a specific first wave number range between 2800 cm-1 and 3000 cm-1 and at a specific second wave number range between 1450 cm⁻¹ and 1710 cm⁻,
- a detector for detecting the intensities of the radiation at first and second specific wave number ranges after it is passed through the sample and
- means for generating a signal related to the detected intensities.

### DETAILED DESCRIPTION OF THE INVENTION

### Method

A method for measuring a biological value Vb in a liver is described herein, wherein said method comprises the steps of:
a/ applying an infrared radiation having at least a first wave number range between 2800 cm-1 and 3000 cm-1 to one or more portions, pi, of a sample of said liver,
b/ detecting the intensity of the radiation after it has passed through each of one or more portions, pᵢ, and generating a signal related to the detected intensity,
c/ processing the generated signal(s) to calculate an average value Vₐ ; by:
   - calculating, for each of the one or more portions, pᵢ, a first value V_{w1pi} related to the first wave number range,
   or
   - calculating the mean of the generated signal(s) for each portion pi to generate an average signal,
   - calculating a first average value V_{w1a} related to the average signal at the first wave number range,
   and calculating an average value Vₐ,
d/ comparing said average value Vₐ to a standard to obtain the biological value Vb.

### Sample of liver

The invention is performed on a solid sample of liver.

The liver may have been removed from an animal, particularly a mammal, for example in order to be grafted. The liver is preferably a human liver.

The sample may be provided from a biopsy of the liver.

The sample may be preserved in standard solution for graft preservation, for example University of Wisconsin solution.

The sample can be prepared preferably by microtomy.

It stands out that the sample is a solid sample and more preferably a tissue frozen section.

For example 20-100 µm thick films can be prepared from a solid sample of liver obtained by freezing the sample.

The sample has a size of preferably 1mmx1mm to 5mmx5mm, more preferably 3mmX3mm to 5mmX5mm, most preferably 5mmX5mm and a thickness between 2µm and 10µm, more preferably between 4µm to 6µm.

The sample may be used immediately or stored until use.

Preferably, before being studied with an infrared spectrometer, the sample is dried a few minutes at room temperature.

The method is non destructive. Thus the sample can be used for further staining, immuno-labeling, spectroscopy or mass spectrometry analysis.

Further, the sample may be heterogeneous.

For example, if the biological value to measure is the level of lipids, the lipids may be non-homogeneously distributed on the whole sample.

Further, once the biopsy has been performed, the sample can be prepared in only a few minutes at room temperature.

### IR spectroscopy and data treatment

The sample is placed in an infrared spectrometer.

The infrared spectrometer that may be used in the method may be any infrared spectrometer commercially available, for example from Bruker Optics, Perkin Elmer, Thermo Scientific, or Varian-Agilent.
In particular, the infrared spectrometer may be a FTIR spectrometer.

The sample used for carrying out the method described herein is divided into one or more portions, usually called pixels.
Each portion corresponds to an area to which an infrared radiation is applied. Preferably, the sample is entirely divided into portions having the same size.
Each portion is preferably between 20µmX20µm and 1000µmX1000µm, more preferably between 50µmX50µm and 500µmX500µm and most preferably about 50µmx50µm.
The choice of the number of portions and the size of the portions depend on the desired time of acquisition.
For example, a sample divided into portions of 100µmX100µm will be analysed four times faster than the same sample divided into portions of 50µmX50µm.

An infrared radiation is applied to a first portion.
The infrared radiation has a first wave number range between 2800 cm-1 and 3000 cm-1.

One can either use wave numbers or the equivalent wavelengths.
For example, wave number ranges of 2800-3000 cm-1 and 1450-1710 cm-1 correspond to wavelengths of 3.57-3.33 micrometers and 6.89-5.85 micrometers, respectively.
The intensity of the radiation after it has passed through the first portion is detected and a signal related to the detected intensity is generated.
For example, the signal may be the spectrum corresponding to absorbance in function of the wave number.
In a first alternative, the step of processing the generated signal(s) to calculate an average value comprises the steps of calculating, for each of the one or more portions, pᵢ, a first value V_{w1pi} related to the first wave number range, and calculating an average value V_{a.}
The signal is then processed to calculate a first value for the first portion (V_{w1p1}).

The integrated intensity of one specific vibrational band is proportional to the quantity of the probed species, as long as the exact pathway of the IR beam through the sample is known (Beer-Lamber law in J. D. J. Ingle and S. R. Crouch, Spectrochemical Analysis, Prentice Hall, New Jersey (1988)).
Thus, for each portion, pᵢ, the calculation of the first value V_{w1pi} may be performed by integrating the intensity of the band corresponding to the first wave number range thereby determining area of peak of the spectra at the first wave number range.
The measure of the intensity and of the area of peak may be related to a reference.
The steps performed on the first portion of the sample are repeated, in the same way, on all the other portions of the sample; thereby one first value V_{w1pi} is calculated for each portion, pᵢ.
Then, an average value Vₐ of the sample may be calculated based on the first values of all the portions.
Preferably, all the portions have the same size.
Preferably, first values V_{w1pi} corresponding to outliers are rejected (artifacts, blood vessels, liver damages during biopsy).
Thus, the mean value of the remaining first values V_{w1pi} is calculated.
In this first alternative, Vₐ may be equal to the mean of the V_{w1pi} or to the mean of the V_{w1pi} normalized where V_{w1pi} of outliers have been rejected or not.
This average value Vₐ is compared to a standard to obtain the biological value Vb.
The standard may be a value, a set of values, a standard curve or calibration curve.
Preferably, the standard is a standard curve or calibration curve.
For example, when the biological value V_{b} is a level of lipids, the standard is a standard curve of average values Vₐ of control samples in function of the concentration of lipids obtained by extraction and quantitative analysis (e.g. using gas-phase chromatography- mass spectrometry) of these control samples.

In a second alternative, the step of processing the generated signal(s) to calculate an average value Vₐ comprises the steps of
- calculating the mean of the generated signal(s) for each portion pi to generate an average signal,
- calculating a first average value V_{w1a} related to the average signal at the first wave number range,
and
- calculating an average value Vₐ,

The generated signals for each portion, pi, are added to generate a total signal that is divided by the number of portions,
Preferably, the generated signals for pi that corresponds to outliers are rejected (artifacts, blood vessels, liver damages during biopsy).
Then, the average signal is processed to calculate an average value V_{w1a} related to the average signal at the first wave number range.
The processing of the average signal is performed in the same way that it was implemented for a single signal of a portion pi.
Thus, for the average signal, the intensity of the band corresponding to the first wave number range thereby determining area of peak of the spectra at the first wave number range.
Then, an average value Vₐ of the sample may be calculated based on the average value at first wave number range V_{w1a}.
This alternative allows to lower the number of calculations and to measure the biological value faster.

In preferred conditions, the infrared radiation has further a second wave number range between 1450 cm⁻¹ and 1710 cm⁻¹.
In these preferred conditions, the step of processing the generated signal(s) to calculate an average value Vₐ further comprises the steps of:
- for each of one or more portions, pᵢ, calculating a second value V_{w2pi} related to the second wave number range in a first alternative,
   or
- calculating the mean of the generated signals for each portion pi to generate an average signal,
- calculating a second average value V_{w2a} related to the average signal at the second wave number range, in a second alternative,
The average value Vₐ is the average ratio V_{w1} / V_{w2}.
In the first alternative, the calculation of the second values V_{w2pi} is performed in the second wave number range in the same way that it was implemented for the first value V_{w1pi} in the first wave number range.
Thus, for each portion, pᵢ, the calculation of the second value V_{w2pi} may be performed by integrating the intensity of the band corresponding to the second wave number range thereby determining area of peak of the spectra at the second wave number range.

Then, in these preferred conditions, the average ratio V_{w1} / V_{w2} is calculated.

The average ratio V_{w1} / V_{w2} may be the arithmetic mean of the individual V_{w1pi} / V_{w2pi} ratios, calculated for each portion pi of the sample.

Alternatively, the average ratio may be calculated by dividing the average first value (arithmetic mean of the individual V_{w1pi} for each portion pi of the sample) by the average second value (arithmetic mean of the individual V_{w2pi} of the sample).

In the second alternative, the calculation of the second average value V_{w2a} is performed in the second wave number range in the same way that it was implemented for the first average value V_{w1a} in the first wave number range.

Then, in these preferred conditions, the average ratio V_{w1} / V_{w2} is calculated.
In this second alternative, the average ratio V_{w1} / V_{w2} is the ratio V_{w1a}/ V_{w2a}.

The second wave number range may be between 1450-1575cm⁻¹.
This wave number range is related to Amides II.
Alternatively, the second wave number range may be between 1660cm-1 and 1710cm-1.
This wave number range is related to Amides I.
The second wave number range may also be between 1450cm-1 and 1575cm-1 together with between 1660cm-1 and 1710cm-1. Preferably, the biological value Vb is a level of lipids, for example the level of triglycerides.

Indeed, without being bound by theory, the wave number range between 2800 cm-1 and 3000 cm-1 is assigned to the chemical functions -CH3 and -CH2 which are mostly related to the contribution of the long carbon chains of lipids.
Thus, analysing the sample at this wave number allows measuring the level of lipids of the sample and in particular the level of triglycerides.
The inventors have shown that this wave number range is particularly relevant for measuring the level of lipids and in particular the level of triglycerides. Indeed, the inventors have shown that the average value of a sample calculated based on the infrared analysis at this wave number range are directly correlated to the level of lipids of this sample measured by extraction of lipids and chromatographic assay with a very good correlation coefficient, as evidenced in the experimental section.
Further, the method described herein allows the use of a sample with high salinity.

### Diagnosis or prognosis of liver diseases

The methods described herein have very valuable qualities.

They allow assessment of biological values Vb of a liver, such as the level of lipids, and more particularly the level of triglycerides.

Therefore, they may be used in particular for diagnosing or prognosing a number of liver diseases.

In particular, the methods described herein may be used for the *in vitro* diagnosis of a fatty liver.
To this end, a biological value Vb, preferably the level of lipids, in a liver is measured according to the described method and it is compared to a threshold value.
A biological value Vb superior to the threshold value is indicative of a fatty liver. The fatty liver is preferably a steatotic liver, for example a liver with a NASH, a liver with steatofibrosis, a liver with hepatocarcinoma, or a liver with cirrhosis.
The method described herein may also be used for determining the level of steatosis of a liver.
To this end, a biological value Vb, preferably the level of lipids, of a liver is measured according to the described method and it is compared to a standard.

The standard may be a lipid concentration, in particular triglycerides, measured from biopsies of steatotic livers.
The standard may also be a lipid concentration, in particular triglycerides, measured from biopsies of liver grafts.
Then, the biological value Vb, preferably the level of lipids, is compared to several threshold values and a biological value Vb between a first threshold value and a second threshold value is indicative of a mild steatosis, a biological value Vb between the second threshold value and a third threshold value is indicative of a moderate steatosis, a biological value Vb superior to the third threshold value is indicative of a severe steatosis.

Another use of the method described herein is for prognosing steatofibrosis, hepatocarcinoma or cirrhosis.
To this end, a biological value Vb, preferably the level of lipids, in a liver is measured according to the method described herein and it is compared to threshold values.
A biological value Vb superior to a first threshold value is indicative of a high risk to develop steatofibrosis,
A biological value Vb superior to a second threshold value is indicative of a high risk to develop hepatocarcinoma,
A biological value Vb superior to a third threshold value is indicative of a high risk to develop cirrhosis.

The method described herein may also be used for determining if a liver is suitable to be grafted.
To this end, the sample provided is a sample of a liver graft and the biological value Vb, preferably the level of lipids, in a liver is measured according to the method described herein and it is compared to threshold values.
A biological value Vb inferior to a first threshold value is indicative that the liver is suitable to be grafted with a poor risk of non-function.
A biological value Vb between the first and a second threshold value is indicative that the liver is suitable to be grafted with a moderate risk of non-function,

A biological value Vb superior to a third threshold value is indicative that the liver is not suitable to be grafted.
A liver with a steatotic level superior to 60% is unsuitable to be grafted.
A liver with a steatotic level between 30%-60% is suitable to be grafted but present a risk of non-function.

A liver with a steatotic level inferior to 30% is suitable to be grafted and presents a poor risk of non-function.
For these various applications, the method described herein may be coupled with a qualitative method such as a histological method or qualitative spectroscopy to provide a more complete diagnosis or prognosis.

The assessment of the biological value Vb according to a method described herein is quantitative, objective and does not depend on the interpretation of a physician.
The method described herein is rapid. It takes less than 15 minutes to measure the biological value Vb, whereas measuring a biological value Vb by extraction of a compound and analysis by chromatography can last 2 days.
The rapidity to determine if a liver is suitable to be grafted is crucial. Indeed, a liver graft from a donor is available only for 15 hours. This period of time includes the time of transportation and preparation. Therefore, the decision to graft a liver has to be taken very quickly. The method is easy to implement even in hospital and is inexpensive. A conventional IR spectrometer can be used.

The method is adapted to the conditions used for preparing a graft, in particular the conditions of salinity of preservative solutions.
The method described herein is very sensitive.
The sensitivity of the method is about 10⁻⁴ M or 10⁻¹² g of molecule.
At the wave number ranges used in the method described herein there is only a poor dispersion, in contrast to other wave number ranges, such as those used as in near infrared spectroscopy.
Furthermore, there is no combination of many chemical functions.

The inventors have shown that the level of steatosis is well correlated with the biological value Vb measured according to the method described herein whether the level of steatosis is high or low.
Because of the small size of the vacuoles in microsteatosis, microsteatosis is often not badly diagnosed with histological methods.
The method described herein being a quantitative method, the results do not depend on the size of vacuoles.
Thus, the method is well adapted in the case of macrosteatosis, exhibiting large vacuoles, as well as in the case of microsteatosis.
Furthermore, the wave number range between 1450 cm-1 and 1710 cm-1 is assigned to the energy domain of Amide I or Amide II corresponding to the vibration(s) of amides, in particular peptidic links in proteins and consequently related to the level of proteins.
The inventors have shown that the calculation of the ratio first value related to lipids/second value related to proteins leads to normalize the intensity thus avoiding variations related to local variations in the thickness of the sample.

### Apparatus

The Inventors additionally designed a new apparatus for implementing the above process.
Therefore, is also described herein an apparatus for measuring a biological value Vb in a sample comprising:
- a site for submitting a sample to an infrared radiation,
- an infrared radiation source for providing a beam of infrared radiation having a wave number range between 1450 cm-1 and 3000 cm-1,
- a detector for detecting the intensities of the radiation at a first specific wave number range between 2800cm-1 and 3000cm-1 and at a second specific wave number range between 1450cm-1 and 1710cm-1 after it is passed through the sample and
- means for generating a signal related to the detected intensities.
The site for submitting a sample to an infrared radiation is suitable to solid sample.
The site for submitting the sample to an infrared radiation may further comprise a mask to adjust the area to which the radiation is provided to the sample.

Preferably only one radiation is provided to the not masked area so that there is only one acquisition for the two frequency regions of interest.
Alternatively, the infrared radiation may be restricted to the first specific wave number range and the second specific wave number range.
The specific second wave number range may be between 1450 cm-1 and 1575cm-1.
Alternatively, the second wave number range may be between 1660cm-1 and 1710cm-1.
The second wave number range may also be between 1450cm-1 and 1575cm-1 together with between 1660cm-1 and 1710cm-1.
The restriction of infrared radiation to specific wave number ranges may be done using a bandpass filter.
The detector may be, for example, a multichannel detector, a liquid nitrogen cooled detector or a room temperature detector.
The apparatus described herein may further comprise an interferometer.

The apparatus uses only a specific and restricted wave number and not all the infrared wave number range. Thus, it is easier to produce, and less expensive.
If an interferometer is used, there is only a need for a small interferometer, easier to produce and less expensive.

Under preferred conditions, the apparatus further comprises:
- a standard and
- a processor for processing the generated signal to calculate:
- a first value V_{w1} obtained by using the first specific wave number range,
- a second value V_{w2} obtained by using the specific second wave number range, and
- the ratio V_{w1}/V_{w2} and
for comparing the ratio V_{w1}/V_{w2} to a standard to obtain the biological value Vb.

The apparatus described herein is suitable for macroscopic analysis.
Usual IR apparatus are suitable to microscopic analysis. To this aim, these apparatus are coupled to a microscope to target a specific area of interest.

Generally, this area responds to morphologic criteria. These apparatus do not analyze the whole sample but only a microscopic part.
The Inventors have found that with a method described herein, the analysis of the sample may be done at the macroscopic level, which allows the quantitative measure of a biological value of a sample.
For such uses, the apparatus is not coupled with a microscope.
A dedicated reflection set up will be inserted in any commercial Fourier Transform Spectrometer.
Further, the apparatus may comprise a beam called macrobeam having a size between 100µmX100µm and 1mmX1mm, more preferably between 500µmX500µm and 1mmX1mm, most preferably of 1mmX1mm.
The macrobeam may be restricted by an aperture if necessary.
The sample manipulation will be simpler, and will take a few seconds instead of the several minutes in the microscopic mode. The sample will need to be deposited on metal-coated glass slides, which are the sample holder

All the processing of signal is done by the apparatus so it is easy to manipulate by any unqualified individual.

The apparatus may also comprise a memory device such as values calculated by the processor and standard values.
Thus, the apparatus may memorize the first values and the second values for portions of the sample and then the processor may use these values to calculate an average value.

### FIGURES

Figure 1 shows histological estimation of steatosis and lipid content on various samples. Steatosis estimated on stained tissue section after HES was plotted as a function of the concentration of triglycerides (TG). A) Macrovacular steatosis. B) Macrovacuolar and microvesicular steatosis.
Figure 2 shows a IR spectra by [(2800-3100)/(1485-1595)].
Figure 3 shows the ratio lipids/proteins calculated from IR spectra that was plotted as a function of the concentration of TG for various samples. The resulting curve may be used as a standard.
Figure 4 shows the comparison between the "Average Ratio" (AR) method and the "Average Spectra" (AS) method.

### EXAMPLES

### Materials and methods

### Patients and liver samples

Liver specimens were obtained from the Centre de Ressources Biologiques Paris-Sud, Paris-Sud XI University, France. Tissue samples were obtained from the non-tumoral part of 27 liver resection specimens. For all patients, daily alcohol consumption was lower than 30 g for men and 20 g for women. Infection with hepatitis B virus (HBV) or hepatitis C virus (HCV), genetic hemochromatosis, autoimmune liver diseases, Wilson's disease were excluded. For routine pathological assessment, tissues were fixed in formalin and one specimen of non-tumorous liver distant to tumor was immediately snap frozen in liquid nitrogen and stored at -80°C until use.
For 6 patients, liver was histologically normal. For 21 other patients, microscopic analysis revealed bland macrovesicular and microvesicular steatosis without hepatocyte ballooning, lobular inflammation, perisinusoidal fibrosis, nor Mallory's hyaline.
For assessment by infrared spectroscopy, the biopsies were immediately frozen in liquid nitrogen and stored at -80°C.

### Lipid profiling

The lipidomic analysis was performed on the platform MetaToul at IFR150 (Toulouse, France). Liver biopsies (5-10mg) were homogenized in 2ml of methanol/ 5mM EGTA (2:1 v/v) with FAST-PREP® (MP Biochemicals). The equivalent of 0.5mg of tissue were evaporated, the dry pellets were dissolved in 0.25 ml of NaOH (0.1M) overnight and proteins were measured with the Bio-Rad assay.

Triglyceride (TG) assays were performed as described in Rebouissou et al., 2007. Briefly, lipids corresponding to an equivalent of 1 mg of tissue were extracted according to Bligh and Dyer in dichloromethane/methanol/water (2.5:2.5:2.1, v/v/v) (Bligh and Dyer, 1959), in the presence of 15 µg of glyceryl triheptadecanoate as an internal standard. Dichloromethane phase was evaporated to dryness, and dissolved in 20 µl of ethyl acetate. 1µl of the lipid extract was analyzed by gas-liquid chromatography on a FOCUS Thermo Electron system using an Zebron-1 Phenomenex® fused silica capillary columns (5m X 0,32mm i.d, 0.50 µm film thickness) (Barrans et al., 1994). Oven temperature was programmed from 200°C to 350°C at a rate of 5°C per minute and the carrier gas was hydrogen (0.5 bar). The injector and the detector were at 315°C and 345°C, respectively.

### Tissue section

Serial sections were 1 to 5mm and cut with 4-6 µm thick at -20°C with a CM3050-S cryostat (Leica Microsystèmes SAS, France) and alternately deposited on glass slide for extemporaneous histological control and on mirrlR gold coated glass slide (Tientascience, Indianapolis, IN) for FTIR microspectroscopy.
Sections for histology were stained with hematoxylin eosin saffron (HES). Sections for FTIR microspectroscopy were dried a few min at room temperature.

### FTIR microspectroscopy and data treatment

Infrared microspectroscopy was performed on IN10MX microscope (ThermoFisher scientific).
All spectra were collected by ultra-fast mode using 50 µm x 50 µm aperture. The spectra were collected in the 4000 - 800 cm-1 mid-infrared range at a resolution of 16 cm-1 with 1 spectrum per pixel.
Data analysis of IR spectra and chemical images were performed using OMNIC software ® (ThermoFisher scientific).

### Results

### Example 1

### Histological estimation of liver steatosis is poorly correlated to the lipid content.

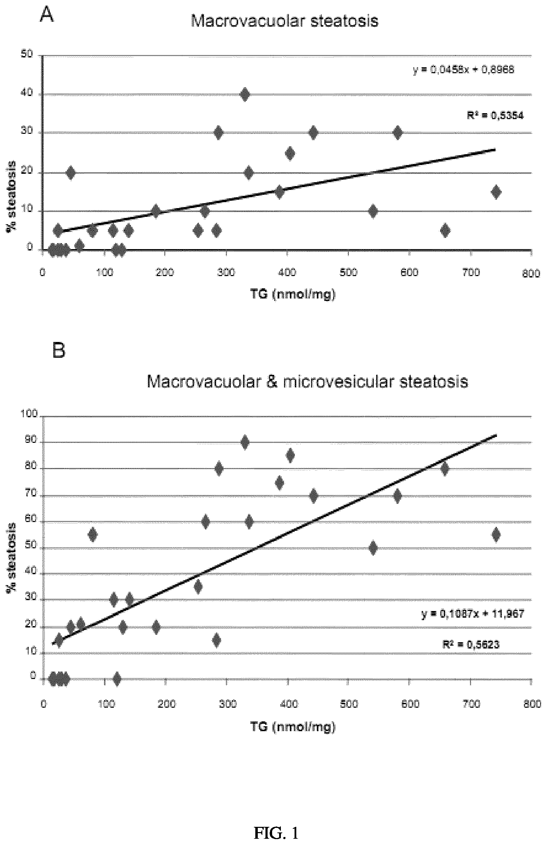

The hallmark feature of steatosis is the intra-cellular accumulation of triacylglycerol (TAG) resulting in the formation of vesicles in the hepatocytes. Therefore the estimation of steatosis is based on histological examination of the number of steatotic cells and the size of steatotic vesicles on tissue sections after H&E staining. This estimation is considered to represent the level of steatosis. However, the correlation between the histological estimation of steatosis and the real lipid content has not been investigated. The lipid content was extracted from 27 human liver biopsies exhibiting various levels of macrovacuolar and microvesicular steatosis ranking between 0-90%. The triglycerides (TG) were quantified by lipidomic analysis using gas phase chromatography coupled to mass spectrometry (GC-MS). The percentage of steatosis was plotted as a function of the concentration of TG (Figure 1). The study was first focused on macrovacuolar steatosis. Important discrepancies were observed between the histological estimation of steatosis and the concentration of TG obtained on the adjacent biopsy for each patient. For instance, low steatosis such as 5% was observed to correspond to a very broad lipid content ranking between 25-658 nmol/mg. For a given concentration of TG such as 300-400 nmol/mg, huge variations were also observed in the estimation of steatosis. Indeed, macrosteatosis was estimated to be 5% to 40% for this range of TG concentrations. Interestingly, the patient exhibiting the highest lipid content with 5% macrosteatosis was also observed to exhibit a high level of microsteatosis 75% demonstrating that the microsteatosis can contribute dramatically to the lipid content. Thus the study was further focused on the complete estimation of steatosis combining macro- and microsteatosis. Important discrepancies were also observed between the histological estimation of steatosis and the concentration of TG.
Results are shown in Table 1 below.
These observations demonstrate that the histological estimation of steatosis is poorly correlated with the lipid content.

### Quantitative assessment of lipid content by infrared spectroscopy

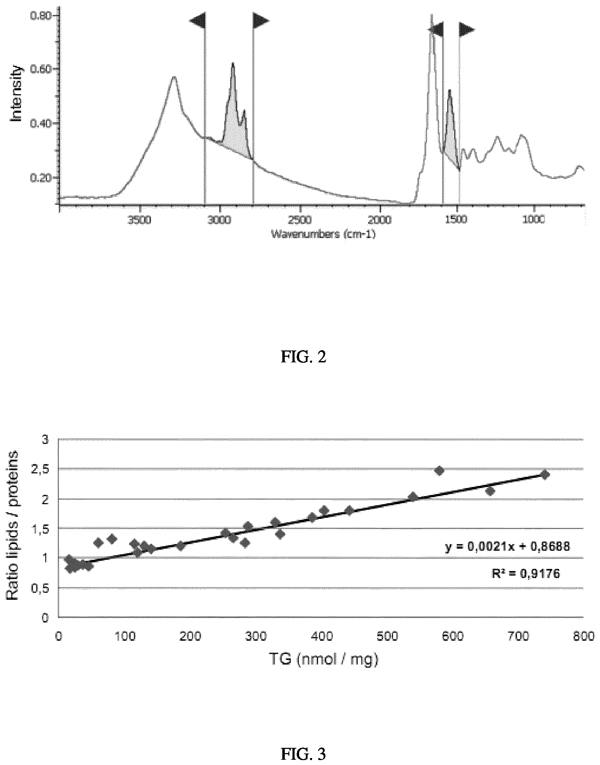

The possibility to address the lipid content directly on tissue section was investigated using infrared microspectroscopy. Thus, infrared microspectroscopy acquisitions were performed on tissue sections from human liver biopsies exhibiting various levels of steatosis. Serial tissue sections were performed using frozen biopsies. Some tissue sections were used for HES staining whereas others were used for spectroscopy experiments. The acquisition of IR spectra were realized using 50 µm x 50 µm aperture size with 1 second for acquisition time. The use of a multi-array detector allowed working 16 times faster than using a single detector. This configuration allows to investigate whole tissue sections in a very short time. The size of tissue sections was ranking between 1 to 5 mm. Thus, scanning 1 mm² to 5 mm² corresponding up to 10 000 spectra was always performed in less than 10 minutes.
Major changes were observed in the lipid frequency domains, such as the relative intensity of the -CH3 and -CH2 (3000-2800 cm-1), olefin (C=C, 3000-3060 cm-1) and of the ester signals (C=O, 1740 cm-1) which increased significantly with the level of steatosis whereas the bands corresponding to proteins, characterized by Amide I and II bands centred respectively at 1650 and 1540 cm-1, were similar (figure 2).
The abundance of lipids related to proteins was further investigated by calculating the ratio lipids/proteins [(2800-3100)/(1485-1595 cm⁻¹)] for each IR spectrum on a large map of a liver section. This calculation leads to normalizing the intensity on every single pixel thus avoiding variations related to local variations in the thickness of the tissue section. Average ratio of lipids/proteins was further obtained from the mean of the all pixels analyzed. The relation between the lipid content within the tissue samples as measured by the FTIR spectroscopy has been calculated and compared to the related amount of TG obtained after lipid extraction and quantitation. The average value for each sample has been plotted as a function of the TG value obtained from lipidomic analysis.

Results are shown in Table 1 below.

There is a marked trend for linearity between the Lipids/Proteins ratio with a correlation coefficient r2= 0.92 (Figure 3). The observed linearity is holding a huge promise to make IR microscopy an efficient diagnostic tool for the steatosis content diagnosis.

**Table 1: Summary of results**

| **Patient #** | **% Macro** | **% micro** | **Cumul Macro & micro** | **TG total** | **Ratio lip/pro t** |
|---|---|---|---|---|---|
| 1 | 0 | 0 | 0 | 17,97 | 0.832 |
| 2 | 0 | 0 | 0 | 30,51 | 0.876 |
| 3 | 5 | 30 | 35 | 254,49 | 1.41 |
| 4 | 10 | 10 | 20 | 185,75 | 1.2 |
| 5 | 30 | 40 | 70 | 580,52 | 2.48 |
| 6 | 30 | 40 | 70 | 443,77 | 1.79 |
| 7 | 30 | 50 | 80 | 288,34 | 1.53 |
| 8 | 20 | 40 | 60 | 337,65 | 1.4 |
| 9 | 0 | 20 | 20 | 130,37 | 1.2 |
| 10 | 0 | 0 | 0 | 37,68 | 0.885 |
| 11 | 0 | 0 | 0 | 15,68 | 0.97 |
| 12 | 1 | 20 | 21 | 61,38 | 1.26 |
| 13 | 5 | 25 | 30 | 115,51 | 1.23 |
| 14 | 5 | 10 | 15 | 25,48 | 0.838 |
| 15 | 0 | 5 | 5 | 120,68 | 1.08 |
| 16 | 0 | 0 | 0 | 25,77 | 0.913 |
| 17 | 40 | 50 | 90 | 331,02 | 1.6 |
| 18 | 5 | 25 | 30 | 141,32 | 1.15 |
| 19 | 5 | 50 | 55 | 81,38 | 1.32 |
| 20 | 15 | 60 | 75 | 386,82 | 1.68 |
| 21 | 25 | 60 | 85 | 405,11 | 1.79 |
| 22 | 10 | 50 | 60 | 266,28 | 1.33 |
| 23 | 15 | 40 | 55 | 741,88 | 2.41 |
| 24 | 20 | 0 | 20 | 45,74 | 0.856 |
| 25 | 5 | 75 | 80 | 658,93 | 2.12 |
| 26 | 10 | 40 | 50 | 541,22 | 2.03 |
| 27 | 5 | 10 | 15 | 284;65 | 1.26 |

### Example 2

### Comparison between two alternative methods

Two alternatives of a method for measuring the lipid content as described herein were compared.

The first one is the method described in the example above. In this method, the ratio lipids/proteins is calculated for each pixel. The pixels are 50µmX50µm. Therefore, to analyse a 500µmX500µm section, 100 pixels are used and 100 ratio lipids/proteins are calculated. Then, average ratio of lipids/proteins is further obtained from the mean of the all pixels analyzed. This method is called below "Average Ratio" (AR) method.
In the second method, the average spectra of 100 pixels is calculated. The ratio lipids/proteins is calculated on the base of the average spectra.
This method is called below "Average Spectra" (AS) method.

19 steatotic patients, including the patient analyzed in example 1, were analyzed.

The study was carried for each patient individually by choosing 4 independent areas on a tissue section.

Patients were categorized in three groups depending on their level of triglycerides (TG).

| Group | | Number of patients |
|---|---|---|
| 1 healthy | [TG] <40 nmol/mg | 5 |
| 2 slightly steatotic | 40 nmol/mg <[TG] < 200 nmol/mg | 5 |
| 3 steatotic | 200 nmol/mg <[TG] < 500 nmol/mg | 5 |
| 4 very steatotic | [TG] > 500 nmol/mg | 4 |

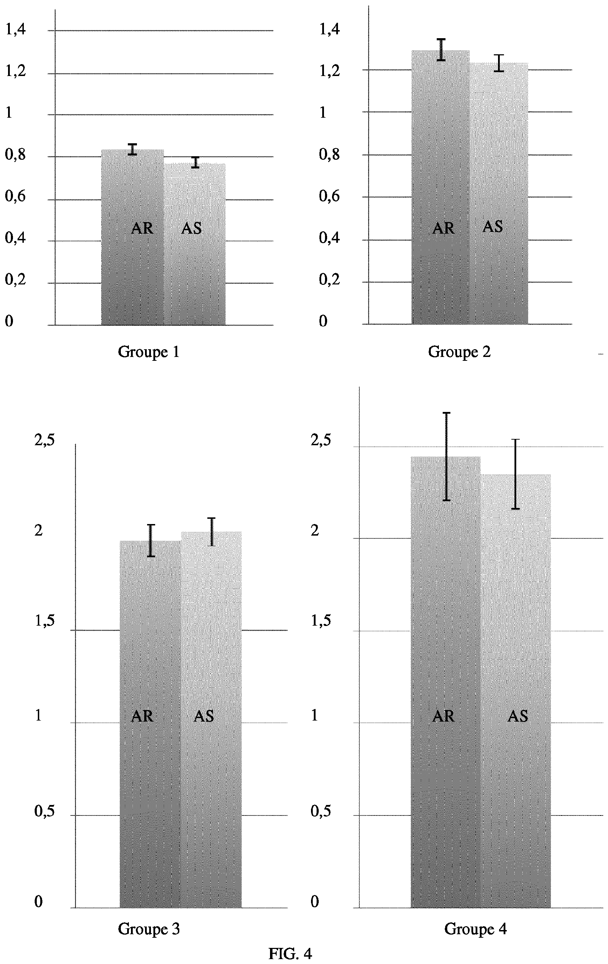

Similar biological values are obtained (see figure 4).
Therefore, an apparatus can analyse a section of tissue with pixels of 500µmX500µm allowing a fast acquisition of IR spectra and measure of the content of lipids.

### REFERENCES

Throughout this application, various references describe the state of the art to which this invention pertains.
Beer-Lamber law in J. D. J. Ingle and S. R. Crouch, Spectrochemical Analysis, Prentice Hall, New Jersey (1988)
Briceno J, Ciria R, Pleguezuelo M, de la Mata M,Muntane J, Naranjo A, Sanchez-Hidalgo J, Marchal T, Rufian S, Lopez-Cillero P Outcome and Viral Recurrence After Liver Transplantation for Hepatitis C Virus Cirrhosis. Liver Transplant 2009; 15:37-48,
Briceno J, Ciria R, , de la Mata M , Rufian S, Lopez-Cillero P Prediction of Graft Dysfunction Based on Extended Criteria Donors in the Model for End-Stage Liver Disease Score Era Transplantation 2010;90: 530-539
El-Badry AM, Breitenstein S, Jochum W, Washington K, Paradis V, Rubbia-Brandt L, Puhan MA, Slankamenac K, Graf R, Clavien PA. Assessment of hepatic steatosis by expert pathologists: the end of a gold standard. Ann Surg. 2009, 250:691-7.
Le Naour F., Bralet M.P., Debois D., Sandt C., Guettier C., Dumas P., Brunelle A., Laprévote O.: Chemical imaging on liver steatosis using synchrotron infrared and Tof-SIMS microspectroscopies. PLoS ONE, 2009, 4:e7408.
Rebouissou S, Imbeaud S, Balabaud C, Boulanger V, Bertrand-Michel J, Tercé F, Auffray C, Bioulac-Sage P, Zucman-Rossi J. HNF1alpha inactivation promotes lipogenesis in human hepatocellular adenoma independently of SREBP-1 and carbohydrate-response element-binding protein (ChREBP) activation. J Biol Chem. 2007, 282: 14437-14446.

## Claims

1. A method for measuring a level of lipids Vb in a liver wherein said method comprises the steps of:
a/ dividing a solid sample of liver into two or more portions, pᵢ,
b/ utilizing an infrared spectrometer to apply an infrared radiation having at least a first wave number range between 2800 cm⁻¹ and 3000 cm⁻¹ to each of the two or more portions, pᵢ, of said sample of said liver,
c/ detecting the intensity of the radiation after it has passed through each of the two or more portions, pᵢ, and generating a signal related to each detected intensity,
d/ processing the generated signals to calculate an average value Vₐ; by:
- calculating, for each of the two or more portions, pᵢ, a first value V_{w1pi} related to the first wave number range, and
- calculating the average value Vₐ based on the first values of all the portions,
or
- calculating the mean of the generated signals for each portion pᵢ to generate an average signal, and
- calculating a first average value V_{w1a} related to the average signal at the first wave number range, wherein V_{w1a} is equal to the average value Vₐ, and
e/ comparing said average value Vₐ to a standard to obtain the level of lipids Vb.

2. The method according to claim 1, wherein the step of processing the generated signals to calculate an average value comprises the steps of:
- calculating, for each of the two or more portions, pᵢ, a first value V_{w1pi} related to the first wave number range, and
- calculating an average value Vₐ based on the first values of all the portions.

3. The method according to claim 1 or claim 2, wherein the steps of processing the generated signals to calculate an average value comprises the steps of:
- calculating the mean of the generated signals for each portion pᵢ to generate an average signal, and
- calculating a first value V_{w1a} related to the average signal at the first wave number range, wherein Vw1a is equal to the average value Va.

4. The method according to any one of claims 1 to 3, wherein:
- the infrared radiation has further a second wave number range between 1450 cm⁻¹ and 1710 cm⁻¹, and
- the steps of processing the generated signals to calculate an average value Vₐ further comprises the steps of:
- for each of the two or more portions, pi, calculating a second value V_{w2pi} related to the second wave number range, wherein the average value Va is the average ratio V_{w1pi}/V_{w2pi},
or
- calculating the mean of the generated signals for each portion pi to generate an average signal,
- calculating a second average value V_{w2a} related to the average signal at the second wave number range, wherein the average value Va is the average ratio V_{w1a}/V_{w2a}

5. The method according to claim 4, wherein the step of processing the generated signals to calculate an average value Vₐ comprises the steps of:
- calculating for each of the two or more portions, pi, a second value V_{w2pi} related to the second wave number range.

6. The method according to claim 4, wherein the step of processing the generated signals to calculate an average value Vₐ comprises steps of:
- calculating the mean of the generated signals for each portion pi to generate an average signal,
- calculating a second average value V_{w2a} related to the average signal at the second wave number range.

7. The method according to any one of claims 4 to 6, wherein the second wave number range is between 1450 cm⁻¹ and 1575 cm⁻¹.

8. The method according to any one of claims 4 to 6, wherein the second wave number range is between 1660 cm⁻¹ and 1710 cm⁻¹.

9. An *in vitro* method for diagnosing a fatty liver comprising the steps of:
- measuring a level of lipids Vb according the method according to any of claims 1 to 8, and
- comparing the level of lipids Vb to threshold values,
wherein a level of lipids Vb superior to the threshold value is indicative of a fatty liver.

10. An *in vitro* method for determining the level of steatosis of a liver comprising the steps of:
- measuring a level of lipids Vb according the method according to any one of claims 1 to 8, and
- comparing the level of lipids Vb to threshold values,
wherein
- a level of lipids Vb between a first threshold value and a second threshold value is indicative of a mild steatosis,
- a level of lipids Vb between the second threshold value and a third threshold value is indicative of a moderate steatosis,
- a level of lipids Vb superior to the third threshold value is indicative of a severe steatosis.

11. An *in vitro* method for determining the risk of developing steatofibrosis, hepatocarcinoma or cirrhosis comprising the steps of:
- measuring a level of lipids Vb according the method according to any one of claims 1 to 8, and
- comparing the level of lipids Vb to threshold values,
wherein
- a level of lipids Vb superior to a first threshold value is indicative of a high risk to develop stetofibrosis,
- a level of lipids Vb superior to a second threshold value is indicative of a high risk to develop hepatocarcinoma,
- a level of lipids Vb superior to a third threshold value is indicative of a high risk to develop cirrhosis.

12. An *in vitro* method for determining if a liver is suitable to be grafted comprising the steps of:
- measuring a level of lipids Vb according the method according to any of claims 1 to 8, and
- comparing the level of lipids Vb to threshold values,
wherein
- a level of lipids Vb inferior to a first threshold value is indicative that the liver is suitable to be grafted with a poor risk of non-function,
- a level of lipids Vb between the first and a second threshold value is indicative that the liver is suitable to be grafted with a moderate risk of non-function,
- a level of lipids Vb superior to a third threshold value is indicative that the liver is not suitable to be grated.

13. The method according to any one of claims 1 to 12, wherein the level of lipids Vb is a level of triglycerides.

## Patentansprüche

1. Verfahren zur Messung eines Spiegels von Vb-Lipiden in einer Leber, wobei das Verfahren die Schritte umfasst:
a/ Teilen einer festen Leberprobe in zwei oder mehr Portionen, pᵢ,
b/ Verwenden eines Infrarot-Spektrometers, um eine Infrarot-Strahlung mit mindestens einem ersten Wellenzahlenbereich zwischen 2800 cm⁻¹ und 3000 cm⁻¹ auf jede der zwei oder mehr Portionen, pᵢ, der genannten Probe der genannten Leber aufzubringen,
c/ Bestimmen der Intensität der Strahlung, nachdem sie durch jede der zwei oder mehr Portionen, pᵢ, hindurchgegangen ist und Erzeugen eines Signals, welches zur jeweils bestimmten Intensität zugehörig ist,
d/ Prozessieren der erzeugten Signale, um einen durchschnittlichen Wert Vₐ zu errechnen durch:
- Berechnen eines ersten Werts V_{w1pi}, welcher zu dem ersten Wellenzahlenbereich zugehörig ist, für jede der zwei oder mehr Portionen, pᵢ, und
- Berechnen des durchschnittlichen Werts Vₐ auf Basis der ersten Werte aller Portionen,
oder
- Berechnen des Mittelwerts der erzeugten Signale für jede Portion pᵢ zur Erzeugung eines durchschnittlichen Signals, und
- Berechnen eines ersten durchschnittlichen Wertes V_{w1a}, der zu dem durchschnittlichen Signal im ersten Wellenzahlenbereich zugehörig ist, worin V_{w1a} gleich dem durchschnittlichen Wert Vₐ ist
und
e/ Vergleichen des genannten durchschnittlichen Wertes Vₐ mit einem Standard, um den Spiegel von Vb-Lipiden zu erhalten.

2. Verfahren nach Anspruch 1, worin der Schritt des Prozessierens des erzeugten Signals zur Errechnung eines durchschnittlichen Wertes die Schritte umfasst:
- Berechnen eines ersten Werts V_{w1pi}, der zu dem ersten Wellenzahlenbereich zugehörig ist, für jede der zwei oder mehr Portionen, pᵢ, und
- Berechnen eines durchschnittlichen Werts Vₐ basierend auf den ersten Werten aller Portionen.

3. Verfahren gemäß Anspruch 1 oder Anspruch 2, worin die Schritte des Prozessierens des erzeugten Signals zur Berechnung eines durchschnittlichen Wertes die Schritte umfasst:
- Berechnen des Mittelwerts der erzeugten Signale für jede Portion pᵢ zur Erzeugung eines durchschnittlichen Signals, und
- Berechnen eines ersten Wertes V_{w1a}, welcher zu dem durchschnittlichen Signal bei dem ersten Wellenzahlenbereich zugehörig ist, worin V_{w1a} gleich dem durchschnittlichen Wert Vₐ ist.

4. Verfahren gemäß einem der Ansprüche 1 bis 3, worin:
- die Infrarot-Strahlung einen zweiten Wellenzahlenbereich zwischen 1450 cm⁻¹ und 1710 cm⁻¹ aufweist, und
- wobei die Schritte des Prozessierens der erzeugten Signale zur Berechnung eines durchschnittlichen Wertes Vₐ weiter die Schritte umfassen:
- Berechnen eines zweiten Wertes V_{w2pi}, der zu dem zweiten Wellenzahlenbereich zugehörig ist, für jede der zwei oder mehr Portionen pᵢ, wobei der durchschnittliche Wert Vₐ das durchschnittliche Verhältnis V_{w1pi}/V_{w2pi} ist, oder
- Berechnen des Mittelwerts der erzeugten Signale für jede Portion pᵢ, zur Erzeugung eines durchschnittlichen Signals,
- Berechnen eines zweiten durchschnittlichen Wertes V_{w2a}, der zu dem durchschnittlichen Signal bei dem zweiten Wellenzahlenbereich zugehörig ist, worin der durchschnittliche Wert Vₐ das Verhältnis V_{w1a}/V_{w2a} ist.

5. Verfahren gemäß Anspruch 4, worin der Schritt des Prozessierens des erzeugten Signals zur Berechnung eines durchschnittlichen Werts Vₐ die Schritte umfasst:
- Berechnen eines zweiten Wertes V_{w2pi}, der zu dem zweiten Wellenzahlenbereich zugehörig ist, für jede der zwei oder mehr Portionen, pᵢ.

6. Verfahren gemäß Anspruch 4, worin der Schritt des Prozessierens des erzeugten Signals zur Berechnung eines durchschnittlichen Werts Vₐ die Schritte umfasst:
- Berechnen des Mittelwerts der erzeugten Signale für jede Portion, pᵢ zur Erzeugung eines durchschnittlichen Signals,
- Berechnen eines zweiten durchschnittlichen Werts V_{w2a}, welcher zu dem durchschnittlichen Signal bei dem zweiten Wellenzahlenbereich zugehörig ist.

7. Verfahren gemäß einem der Ansprüche 4 bis 6, worin der zweite Wellenzahlenbereich zwischen 1450 cm⁻¹ und 1575 cm⁻¹ liegt.

8. Verfahren gemäß einem der Ansprüche 4 bis 6, worin der zweite Wellenzahlenbereich zwischen 1660 cm⁻¹ und 1710 cm⁻¹ liegt.

9. *In vitro* Verfahren zur Diagnose einer Fettleber umfassend die Schritte:
- Messen eines Spiegels von Vb-Lipiden durch das Verfahren gemäß einem der Ansprüche 1 bis 8 und
- Vergleichen des Spiegels von Vb-Lipiden zu Grenzwerten,
worin ein Spiegel an Vb-Lipiden, welcher höher als der Grenzwert liegt, eine Fettleber anzeigt.

10. *In vitro* Verfahren zur Bestimmung des Steatose-Zustands einer Leber umfassend die Schritte:
- Messen des Spiegels von Vb-Lipiden durch das Verfahren gemäß einem der Ansprüche 1 bis 8 und
- Vergleichen des Spiegels von Vb-Lipiden mit Grenzwerten,
worin
- ein Spiegel von Vb-Lipiden zwischen einem ersten Grenzwert und einem zweiten Grenzwert eine milde Steatose anzeigt,
- ein Spiegel von Vb-Lipiden zwischen dem zweiten Grenzwert und einem dritten Grenzwert eine moderate Steatose anzeigt,
- ein Spiegel von Vb-Lipiden, welcher höher als der dritte Grenzwert liegt, eine schwere Steatose anzeigt.

11. *In vitro* Verfahren zur Bestimmung des Risikos, eine Steatofibrose, ein Leberkarzinom oder Zirrhose zu entwickeln, umfassend die Schritte:
- Messen des Spiegels von Vb-Lipiden durch das Verfahren gemäß einem der Ansprüche 1 bis 8 und
- Vergleichen des Spiegels von Vb-Lipiden mit Grenzwerten,
worin
- ein Spiegel von Vb-Lipiden, welcher höher als ein erster Grenzwert ist, ein hohes Risiko für die Entwicklung einer Steatofibrose anzeigt,
- ein Spiegel von Vb-Lipiden, welcher höher als ein zweiter Grenzwert ist, ein hohes Risiko der Entwicklung eines Hepatokarzinoms anzeigt,
- ein Spiegel von Vb-Lipiden, welcher höher als ein dritter Grenzwert ist, ein hohes Risiko der Entwicklung einer Zirrhose anzeigt.

12. *In vitro* Verfahren zur Bestimmung, ob eine Leber geeignet für eine Transplantation ist, umfassend die Schritte:
- Messen eines Spiegels von Vb-Lipiden durch das Verfahren gemäß einem der Ansprüche 1 bis 8 und
- Vergleichen des Spiegels von Vb-Lipiden mit Grenzwerten,
worin
- ein Spiegel von Vb-Lipiden, welcher geringer als ein erster Grenzwert ist, anzeigt, dass die Leber für Transplantation mit einem geringen Risiko der Nichtfunktion geeignet ist,
- ein Spiegel von Vb-Lipiden zwischen einem ersten und einem zweiten Grenzwert anzeigt, dass die Leber mit einem moderaten Risiko der Nichtfunktion zur Transplantation geeignet ist,
- ein Spiegel von Vb-Lipiden oberhalb eines dritten Grenzwerts anzeigt, dass die Leber nicht geeignet für eine Transplantation ist.

13. Verfahren gemäß einem der Ansprüche 1 bis 12, worin der Spiegel von Vb-Lipiden ein Spiegel von Triglyzeriden ist.

## Revendications

1. Procédé de mesure d'un niveau de lipides Vb dans un foie, dans lequel ledit procédé comprend les étapes de :
a/ division d'un échantillon solide de foie en deux fragments, pᵢ, ou plus
b/ utilisation d'un spectromètre infrarouge pour appliquer un rayonnement infrarouge ayant au moins un premier intervalle de nombre d'ondes compris entre 2 800 cm⁻¹ et 3 000 cm⁻¹ sur chacun des deux fragments, pᵢ, ou plus dudit échantillon dudit foie,
c/ détection de l'intensité du rayonnement après son passage à travers chacun des deux fragments, pᵢ, ou plus et la génération d'un signal associé à chaque intensité détectée,
d/ traitement des signaux générés afin de calculer une valeur moyenne Vₐ ; par :
- calcul, pour chacun des deux fragments, pᵢ, ou plus d'une première valeur V_{w1pi} associée au premier intervalle de nombre d'ondes, et
- calcul de la valeur moyenne Vₐ sur la base des premières valeurs de tous les fragments,
ou
- calcul de la moyenne des signaux générés pour chaque fragment pᵢ pour générer un signal moyen, et
- calcul d'une première valeur V_{w1a} associée au signal moyen au niveau du premier intervalle de nombre d'ondes, dans lequel V_{w1a} est égal à la valeur moyenne Vₐ, et
e/ la comparaison de ladite valeur moyenne Vₐ à un étalon afin d'obtenir le niveau de lipides Vb.

2. Procédé selon la revendication 1, dans lequel l'étape de traitement des signaux générés pour calculer une valeur moyenne comprend les étapes de :
- calcul, pour chacun des deux fragments, pᵢ, ou plus, d'une première valeur V_{w1pi} associée au premier intervalle de nombre d'ondes, et
- calcul d'une valeur moyenne Vₐ sur la base des premières valeurs de tous les fragments.

3. Procédé selon la revendication 1 ou la revendication 2, dans lequel les étapes du traitement des signaux générés pour calculer une valeur moyenne comprennent les étapes de :
- calcul de la moyenne des signaux générés pour chaque fragment pᵢ pour générer un signal moyen, et
- calcul d'une première valeur V_{w1a} associée au signal moyen au niveau du premier intervalle de nombre d'ondes, dans lequel V_{w1a} est égal à la valeur moyenne Va.

4. Procédé selon l'une quelconque des revendications 1 à 3, dans lequel :
- le rayonnement infrarouge présente en outre un second intervalle de nombre d'ondes entre 1 450 cm⁻¹ et 1710 cm⁻¹, et
- les étapes de traitement des signaux générés pour calculer une valeur moyenne Vₐ comprennent en outre les étapes de :
- pour chacun des deux fragments, pᵢ, ou plus, calcul d'une seconde valeur V_{w2pi} associée au second intervalle de nombre d'ondes, dans lequel la valeur moyenne Va est le rapport moyen V_{w1pi}/V_{w2pi}.
ou
- calcul de la moyenne des signaux générés pour chaque fragment pᵢ pour générer un signal moyen,
- calcul d'une seconde valeur moyenne V_{w2a} associée au signal moyen au niveau du second intervalle de nombre d'ondes, dans lequel la valeur moyenne Va est le rapport moyen V_{w1a}/V_{w2a}.

5. Procédé selon la revendication 4, dans lequel l'étape de traitement des signaux générés pour calculer une valeur moyenne Vₐ comprend les étapes de :
- calcul pour chacun des deux fragments, pᵢ, ou plus, d'une seconde valeur V_{w2pi} associée au second intervalle de nombre d'ondes.

6. Procédé selon la revendication 4, dans lequel l'étape de traitement des signaux générés pour calculer une valeur moyenne Vₐ comprend les étapes de :
- calcul de la moyenne des signaux générés pour chaque fragment pᵢ pour générer un signal moyen,
- calcul d'une seconde valeur moyenne V_{w2a} associée au signal moyen au niveau du second intervalle de nombre d'ondes.

7. Procédé selon l'une quelconque des revendications 4 à 6, dans lequel le second intervalle de nombre d'ondes est compris entre 1 450 cm⁻¹ et 1 575 cm⁻¹.

8. Procédé selon l'une quelconque des revendications 4 à 6, dans lequel le second intervalle de nombre d'ondes se trouve entre 1 660 cm⁻¹ et 1 710 cm⁻¹.

9. Procédé *in vitro* de diagnostic de stéatose hépatique comprenant les étapes de :
- mesure d'un niveau de lipides Vb conformément au procédé selon l'une quelconque des revendications 1 à 8, et
- comparaison du niveau de lipides Vb à des valeurs seuils,
dans lequel un niveau de lipides Vb supérieur à la valeur seuil indique une stéatose hépatique.

10. Procédé *in vitro* de détermination du niveau de stéatose hépatique d'un foie comprenant les étapes de :
- mesure d'un niveau de lipides Vb conformément au procédé selon l'une quelconque des revendications 1 à 8, et
- comparaison du niveau de lipides Vb à des valeurs seuils,
dans lequel
- un niveau de lipides Vb compris entre une première valeur seuil et une deuxième valeur seuil indique une stéatose légère,
- un niveau de lipides Vb compris entre une deuxième valeur seuil et une troisième valeur seuil indique une stéatose modérée,
- un niveau de lipides Vb supérieur à la troisième valeur seuil indique une stéatose grave.

11. Procédé *in vitro* de détermination du risque de développement d'une stéatofibrose, d'un hépatocarcinome ou d'une cirrhose comprenant les étapes de :
- mesure d'un niveau de lipides Vb conformément au procédé selon l'une quelconque des revendications 1 à 8, et
- comparaison du niveau de lipides Vb à des valeurs seuils,
dans lequel
- un niveau de lipides Vb supérieur à une première valeur seuil indique un risque élevé de développement d'une stéatofibrose,
- un niveau de lipides Vb supérieur à une deuxième valeur seuil indique un risque élevé de développement d'un hépatocarcinome,
- un niveau de lipides Vb supérieur à une troisième valeur seuil indique un risque élevé de développement d'une cirrhose.

12. Procédé *in vitro* de détermination du fait qu'un foie est adapté ou non à être greffé comprenant les étapes de :
- mesure d'un niveau de lipides Vb conformément au procédé selon l'une quelconque des revendications 1 à 8, et
- comparaison du niveau de lipides Vb à des valeurs seuils,
dans lequel
- un niveau de lipides Vb inférieur à une première valeur seuil indique que le foie est adapté à être greffé avec un faible risque de non-fonction,
- un niveau de lipides Vb compris entre la première valeur seuil et une deuxième valeur seuil indique que le foie est adapté à être greffé avec un risque modéré de non-fonction,
- un niveau de lipides Vb supérieur à une troisième valeur seuil indique que le foie n'est pas adapté à être greffé.

13. Procédé selon l'une quelconque des revendications 1 à 12, dans lequel le niveau de lipides Vb est un niveau de triglycérides.
